# EUROPEAN PATENT SPECIFICATION

(11) **EP 2 886 072 B1**
(45) Date of publication and mention of the grant of the patent: **08.08.2018**
(21) Application number: 14193462.0
(22) Date of filing: 17.11.2014
(51) Int. Cl.: A61B 17/34, A61B 17/11, A61F 2/82

(54) **Catheter for common hepatic duct**
Katheter für gemeinsamen Lebergang
Cathéter pour conduit hépatique commun

(30) Priority: 17.12.2013 KR 20130157385
(43) Date of publication of application: 24.06.2015
(73) Proprietor: Standard Sci-Tech Inc., Seoul 130-823 (KR); Park, Do Hyun, Songpa-gu, Seoul (KR)
(72) Inventor: Park, Do Hyun, Seoul (KR); Jeon, Sang Koo, Gyeonggi-do (KR); An, Sung Soon, Seoul 03493 (KR)
(74) Representative: AWA Sweden AB

(56) References cited:
- US-A1- 2003 120 292
- US-A1- 2008 208 161
- US-A1- 2009 069 822
- US-A1- 2010 324 488
- US-A1- 2012 150 070
- US-A1- 2013 310 833

## Description

### BACKGROUND OF THE INVENTION

### (a) Field of the Invention

The present invention relates to a catheter for a common hepatic duct capable of inserting a stent into a bile duct and the like using endoscopic ultrasound (EUS).

### (b) Description of the Related Art

Generally, a biliary tract provides a passage through which bile is excreted from a liver to an intestine, and controls flow of the bile. The bile moves from left and right hepatic ducts of the liver to a gall bladder. The bile concentrated in the gall bladder is excreted through the bile duct and thus finally flows into the intestine via a common bile duct.

When there is a need to perform a stent insertion due to a lesion of the bile duct and the like, the stent insertion is performed by perforating a stomach and a duodenum using a perforator attached with a needle, inserting a guide wire into the bile duct, and then inserting a stent insertion mechanism thereinto through the guide wire.

The typical stent insertion has a difficulty in expanding a perforated portion of the stomach and the duodenum several times, and thus an operation time is increased and an insertion failure rate of the insertion mechanism is increased, such that it is difficult to perform the stent insertion.

### [Related Art Document]

### [Patent Document]

(Patent Document 1) Korean U.M. Laid-Open Publication No. 20-2011-0001551 (Laid-Open Published on February 15, 2011)

The above information disclosed in this Background section is only for enhancement of understanding of the background of the invention and therefore it may contain information that does not form the prior art that is already known in this country to a person of ordinary skill in the art.

### SUMMARY OF THE INVENTION

The present invention, as described in claim 1, has been made in an effort to provide a catheter for a common hepatic duct capable of reducing an operation time taken to perform a stent insertion in a bile duct, a gall bladder, and the like by perforating a stomach and a duodenum and helping a medical staff simply perform a stent insertion.

An exemplary embodiment of the present invention provides a catheter for a common hepatic duct, including: an inner guide tube; a perforator coupled with a front end of the inner guide tube; a pressing member coupled with the inner guide tube while maintaining a predetermined interval from the perforator; a stent disposed between the perforator and the pressing member and on an outer circumferential surface of the inner guide tube; and an outer guide tube disposed on the outer circumferential surface of the inner guide tube,
wherein the perforator has a cone shape or a polygonal cone shape and is formed so that a distance from a central line to an outside is reduced toward the front end based on the central line in a length direction.

In the perforator, a cross surface taken along a right angle direction with respect to the central line in the length direction may be formed in any one of a circle, a triangle, a pentagon, a hexagon, and an octagon.

In the perforator, a contact portion with a front end of the outer guide tube may be provided with a projection.

The perforator may include: a first inclined portion having an angle reducing from one end of the outer guide tube toward the front end based on the central line in the length direction; and a second inclined portion having an angle reducing from the first inclined portion toward the front end, and an inclined angle of the first inclined portion may be formed to be larger than that of the second inclined portion.

In the perforator, an outer circumferential surface may be provided with a corner portion, and the corner portion may be provided with a cut part protruding along the length direction.

In the cut part, an angle formed by the cut part may range from 30° to 45° when viewed from a cut surface cut in the right angle direction based on the central line in the length direction.

As set forth above, according to an exemplary embodiment of the present invention, it is possible to reduce the operation time of the stent and simply perform the stent operation by allowing the perforator to perforate the stomach and the duodenum and then directly performing the stent insertion in the bile duct, the gall bladder, and the like at the time of performing the stent insertion in the bile duct, the gall bladder, and the like by perforating the stomach and the duodenum.

### BRIEF DESCRIPTION OF THE DRAWINGS

FIG. 1 is a diagram illustrating an overall shape of a catheter for a common hepatic duct for describing an exemplary embodiment of the present invention.
FIG. 2 is a diagram illustrating a perforator which is a main part of FIG. 1.
FIG. 3 is a cross-sectional view taken along the line III-III of FIG. 2.
FIG. 4 is a cross-sectional view taken along the line IV-IV of FIG. 2.
FIG. 5 is a diagram corresponding to FIG. 4 as another example of the exemplary embodiment of the present invention.

### DETAILED DESCRIPTION OF THE EMBODIMENTS

The present invention will be described more fully hereinafter with reference to the accompanying drawings, in which exemplary embodiments of the invention are shown. As those skilled in the art would realize, the described embodiments may be modified in various different ways, all without departing from the scope of the present invention defined in the claims. The drawings and description are to be regarded as illustrative in nature and not restrictive. Like reference numerals designate like elements throughout the specification.

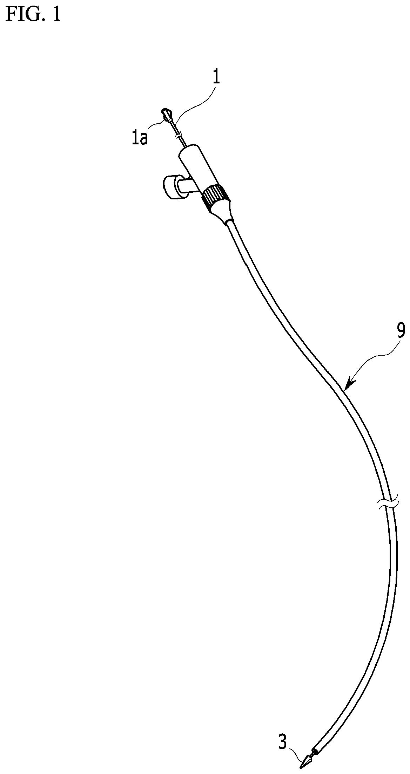

FIG. 1 is a perspective view for describing an exemplary embodiment of the present invention, and illustrates a catheter for a common hepatic duct.

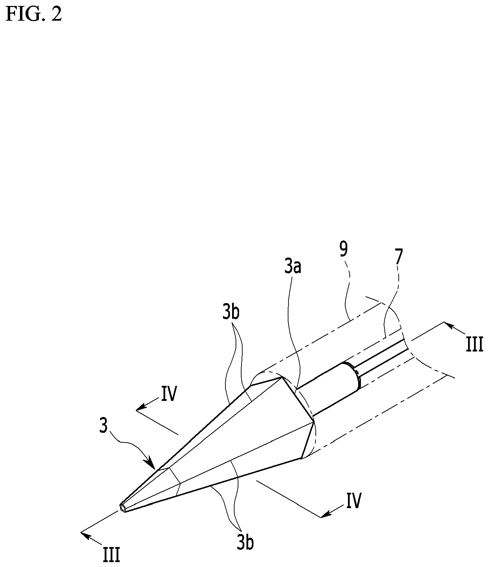

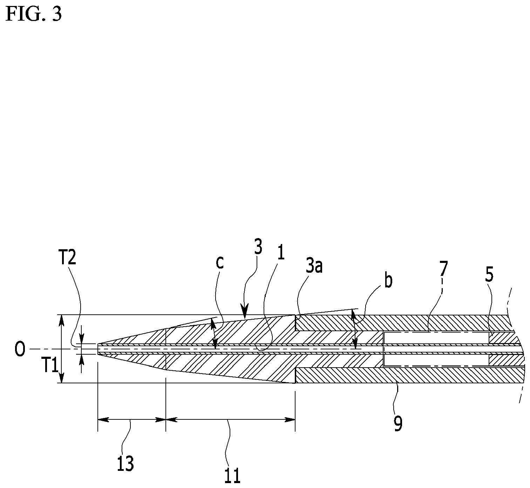

As illustrated in FIGS. 1 to 3, the catheter for a common hepatic duct according to the exemplary embodiment of the present invention includes an inner guide tube 1, a perforator 3, a pressing member 5 (illustrated in FIG. 3), a stent 7 (illustrated in FIG. 3), and an outer guide tube 9.

The inner guide tube 1 has an elongated tube shape, and one end thereof may be coupled with the perforator 3 and the other end thereof may be coupled with a knob 1a. The knob 1a may be gripped by an operator's hand, and thus moves in a length direction of the inner guide tube 1 to be able to move the inner guide tube 1.

The perforator 3 is a part which may perforate a stomach wall and a duodenum wall, and has a shape tapering toward a front end thereof. The perforator 3 may have a circular cone shape or a polygonal cone shape.

The perforator 3 is preferably formed so that a distance (represented by T1 and T2 in FIG. 3) from a central line O to an outside is reduced toward the front end based on the central line O in the length direction (based on a length direction of the inner guide tube or the perforator) (see FIG. 3).

That is, in the perforator 3, a cross surface taken along a right angle direction with respect to the central line O in a length direction is formed as any one of a circle, a triangle, a pentagon, a hexagon, and an octagon.

When the perforator 3 is formed in a triangular, pentagonal, hexagonal, or octagonal cone shape, an inclined corner portion may serve to expand the stomach wall and the duodenum wall at the time of perforating the stomach wall and the duodenum wall.

Further, in the perforator 3, a contact portion with a front end of the outer guide tube 9 is provided with a projection 3a.

Further, according to another example of the exemplary embodiment of the present invention, the perforator 3 may have a first inclined portion 11 up to approximately a one third point of the overall length from the projection 3a (or one end of the outer guide tube) toward the front end, and a second inclined portion 13 from the first inclined portion 11 to the front end (see FIG. 3).

An inclined angle b of the first inclined portion 11 is preferably formed to be smaller than an inclined angle c of the second inclined portion 13 (see FIG. 3).

Meanwhile, the pressing member 5 is coupled with the inner guide tube 1 while maintaining a predetermined interval from the perforator 3. The pressing member 5 may serve to push the stent 7 to the outside of the outer guide tube 9 depending on the movement of the inner guide tube 1.

Further, the stent 7 is disposed between the perforator 3 and the pressing member 5. The stent 7 is disposed on an outer circumferential surface of the inner guide tube 1 and thus is pushed by the pressing member 5 so as to be discharged to the outside of the outer guide tube 9. The stent 7 is inserted into a bile duct and a gall bladder with a lesion and may serve to perforate the bile duct. As the stent 7 inserted in the bile duct and the gall bladder with a lesion, an already known stent may be used, and therefore the detailed description of the stent will be omitted.

The outer guide tube 9 may be formed of an elongated, flexible tube and is disposed on the outer circumferential surface of the inner guide tube 1. One end of the outer guide tube 9 may adhere to the projection 3a of the perforator 3 as described above (see FIG. 1).

The inner guide tube 1, the pressing member 5, and the stent 7 which are described above are preferably disposed inside the outer guide tube 9.

An action of the exemplary embodiment of the present invention configured as described above will now be described in detail.

The catheter for a common hepatic duct according to the exemplary embodiment of the present invention is inserted into a stomach and a duodenum through a working channel using endoscopic ultrasound in the state in which the operator performs an operation using the endoscopic ultrasound. Further, the operator pushes the outer guide tube 9 to perforate the stomach wall and the duodenum wall around the bile duct and the gall bladder. In this case, the perforator 3 has a sharp tip portion, has a structure in which a size of the perforator 3 increases from the front end of the perforator 3 toward a rear end thereof, and has a shape in which a corner is inclined when the perforator 3 is formed as a polygonal pyramid. Therefore, the operator may push the perforator 3 to the stomach wall and the duodenum wall so as to be able to easily perforate the stomach wall and the duodenum wall. Further, the operator may perforate the stomach wall and the duodenum wall with a one-time operation and perforate the stomach wall and the duodenum wall at a sufficient size to pass the outer guide tube 9 therethrough.

The operator then moves the perforator 3 up to the bile duct and the gall bladder into which the stent is inserted. Further, the operator disposes the perforator 3 at the bile duct portion and the gall bladder portion with a lesion and moves the knob 1a in the length direction of the inner guide tube 1.

Then, the pressing member 5 moves while the inner guide tube 1 moves and the pressing member 5 pushes the stent 7. Therefore, the stent 7 exits the outer guide tube 9 and is inserted into the bile duct with a lesion.

After the operator performs insertion of the stent 7 at the lesion, the knob 1a is pulled to receive the inner guide tube 1 in the outer guide tube 9. Then, the projection 3a of the perforator 3 is in close contact with the front end of the outer guide tube 9. The operator then pulls the outer guide tube 9 to remove the catheter for a common hepatic duct from a lumen of a human body.

The catheter for a common hepatic duct according to the exemplary embodiment of the present invention may perforate the stomach wall and the duodenum wall with a one-time operation and then insert the stent 7 in the bile duct and the gall bladder, and as a result, may be conveniently used and remarkably reduce an operation time.

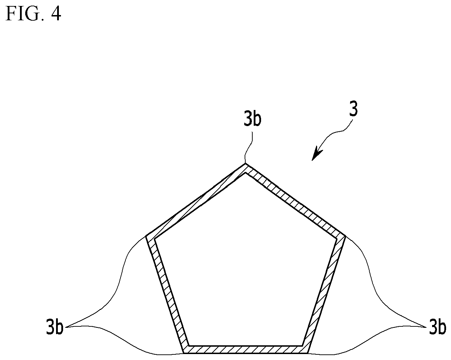

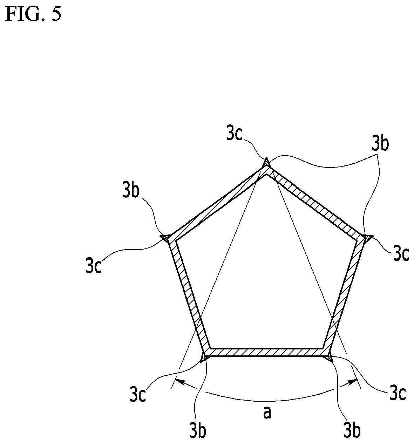

FIG. 5 is a diagram corresponding to FIG. 4 for describing another example of the exemplary embodiment of the present invention, and illustrates a cross-section of the perforator 3.

In another example of the exemplary embodiment of the present invention, the same components as in the above description are replaced by the above description, and only different components from the above description will be described.

A corner portion 3b installed at the outer circumferential side of the perforator 3 having a polygonal cone shape is provided with a cut part 3c protruding along a length direction of the corner portion 3b. In the cut part 3c, an angle a formed by the cut part 3c preferably ranges from 30° to 45°, when viewed from a cut surface cut in a right angle direction based on the central line O in the length direction.

The cut part 3c may serve as the edge of a knife to easily perforate the stomach wall and the duodenum wall when the perforator 3 perforates the stomach wall and the duodenum wall. The cut part 3c may more easily perforate the stomach wall and the duodenum wall.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### <Description of symbols>

| | |
|---|---|
| 1. Inner guide tube | 1a. Knob, |
| 3. Perforator | 3a. Projection |
| 3b. Corner portion | 3c. Cut part |
| 5. Pressing member | 7. Stent |
| 9. Outer guide tube | 11. First inclined portion |
| 13. Second inclined portion | |

## Claims

1. A catheter for a common hepatic duct, **characterized by** comprising:
an inner guide tube (1);
a perforator (3) coupled with a front end of the inner guide tube (1);
a pressing member (5) coupled with the inner guide tube (1) while maintaining a predetermined interval from the perforator (3);
a stent (7) disposed between the perforator (3) and the pressing member (5) and on an outer circumferential surface of the inner guide tube (1); and
an outer guide tube (9) disposed on the outer circumferential surface of the inner guide tube (1),
wherein the perforator (3)
has a cone shape or a polygonal cone shape and is formed so that a distance (T1, T2) from a central line (O) to an outside is reduced toward the front end based on the central line (O) in a length direction.

2. The catheter for a common hepatic duct of claim 1, wherein, in the perforator (3), a cross surface taken along a right angle direction with respect to the central line (O) in the length direction is formed in any one of a circle, a triangle, a pentagon, a hexagon, and an octagon.

3. The catheter for a common hepatic duct of claim 1, wherein, in the perforator (3), a contact portion with a front end of the outer guide tube (9) is provided with a projection (3a).

4. The catheter for a common hepatic duct of claim 1, wherein the perforator (3) includes:
a first inclined portion (11) having an angle that reduces from one end of the outer guide tube (9) toward the front end based on the central line (O) in the length direction; and
a second inclined portion (13) having an angle that reduces from the first inclined portion (11) toward the front end,
wherein an inclined angle of the first inclined portion (11) is formed to be larger than that of the second inclined portion (13).

5. The catheter for a common hepatic duct of claim 1, wherein, in the perforator (3), an outer circumferential surface is provided with a corner portion (3b) and the corner portion (3b) is provided with a cut part (3c) protruding along the length direction.

6. The catheter for a common hepatic duct of claim 5, wherein, in the cut part (3c), an angle formed by the cut part (3c) ranges from 30° to 45° when viewed from a cut surface cut in a right angle direction based on the central line (O) in the length direction.

## Patentansprüche

1. Katheter für einen gemeinsamen Lebergang, **dadurch gekennzeichnet, dass** er umfasst:
ein inneres Führungsrohr (1);
einen Perforator (3), der an ein vorderes Ende des inneren Führungsrohrs (1) koppelt;
ein Druck ausübendes Element (5), das an das innere Führungsrohr (1) koppelt, während ein vorbestimmtes Intervall von dem Perforator (3) gehalten wird;
einen Stent (7), der zwischen dem Perforator (3) und dem Druck ausübenden Element (5) und auf einer äußeren Umfangsfläche des inneren Führungsrohrs (1) angeordnet ist; und
ein äußeres Führungsrohr (9), das auf der äußeren Umfangsfläche des inneren Führungsrohrs (1) angeordnet ist,
wobei der Perforator (3) eine Konusform oder eine vieleckige Konusform aufweist und so geformt ist, dass ein Abstand (T1, T2) von einer Mittellinie (O) zu einer Außenseite in Richtung des vorderen Endes basierend auf der Mittellinie (O) in einer Längsrichtung reduziert ist.

2. Katheter für einen gemeinsamen Lebergang nach Anspruch 1, wobei eine Querschnittfläche, die in dem Perforator (3) entlang einer rechtwinkligen Richtung in Bezug auf die Mittellinie (O) in der Längsrichtung (O) genommen worden ist, in einem beliebigen von einem Kreis, einem Dreieck, einem Fünfeck, einem Sechseck und einem Achteck geformt ist.

3. Katheter für einen gemeinsamen Lebergang nach Anspruch 1, wobei in dem Perforator (3) ein Kontaktabschnitt mit einem vorderen Ende des äußeren Führungsrohrs (9) mit einem Vorsprung (3a) ausgestattet ist.

4. Katheter für einen gemeinsamen Lebergang nach Anspruch 1, wobei der Perforator (3) einschließt:
einen ersten schrägen Abschnitt (11) mit einem Winkel, der von einem Ende des äußeren Führungsrohrs (9) in Richtung des vorderen Endes basierend auf der Mittellinie (O) in Längsrichtung kleiner wird; und
einen zweiten schrägen Abschnitt (13) mit einem Winkel, der von dem ersten schrägen Abschnitt (11) in Richtung des vorderen Endes kleiner wird,
wobei ein schräger Winkel des ersten schrägen Abschnitts (11) so geformt ist, dass er größer als derjenige des zweiten schrägen Abschnitts (13) ist.

5. Katheter für einen gemeinsamen Lebergang nach Anspruch 1, wobei in dem Perforator (3) eine äußere Umfangsfläche mit einem Eckenabschnitt (3b) ausgestattet ist und der Eckenabschnitt (3b) mit einem Schnittteil (3c) ausgestattet ist, das entlang der Längsrichtung hervor ragt.

6. Katheter für einen gemeinsamen Lebergang nach Anspruch 5, wobei in dem Schnittteil (3c) ein durch das Schnittteil (3c) gebildeter Winkel im Bereich von 30° bis 45° liegt, gesehen von einem Schnittflächenschnitt in rechtwinkliger Richtung bezogen auf die Mittellinie (O) in Längsrichtung.

## Revendications

1. Cathéter pour conduit hépatique commun, **caractérisé en ce qu'**il comprend :
un tube de guidage interne (1) ;
un perforateur (3) couplé à une extrémité frontale du tube de guidage interne (1) ;
un élément de compression (5) couplé au tube de guidage interne (1) tout en maintenant un intervalle prédéterminé par rapport au perforateur (3),
un stent (7) disposé entre le perforateur (3) et l'élément de compression (5) et sur une surface circonférentielle extérieure du tube de guidage interne (1) ; et
un tube de guidage externe (9) disposé sur la surface circonférentielle extérieure du tube de guidage interne (1),
le perforateur (3) ayant une forme de cône ou une forme de cône polygonal et étant conformé de manière à ce qu'une distance (T1, T2) entre une ligne centrale (O) et un extérieur soit réduite en direction de l'extrémité frontale par rapport à la ligne centrale (O) dans un sens de la longueur.

2. Cathéter pour conduit hépatique commun selon la revendication 1, dans lequel, dans le perforateur (3), une surface transversale prise suivant un sens à angle droit par rapport à la ligne centrale (O) dans le sens de la longueur est réalisée en une forme quelconque parmi un cercle, un triangle, un pentagone, un hexagone et un octogone.

3. Cathéter pour conduit hépatique commun selon la revendication 1, dans lequel, dans le perforateur (3), une section de contact avec une extrémité frontale du tube de guidage sterne (9) est pourvue d'une saillie (3a).

4. Cathéter pour conduit hépatique commun selon la revendication 1, dans lequel le perforateur (3) comprend :
une première section inclinée (11) décrivant un angle qui diminue depuis une extrémité du tube de guidage externe (9) en direction de l'extrémité frontale par rapport à la ligne centrale (O) dans le sens de la longueur ; et
une seconde section inclinée (13) décrivant un angle qui diminue depuis la première section inclinée (11) en direction de l'extrémité frontale,
un angle incliné de la première section inclinée (11) étant formé de manière à être plus grand que celui de la seconde section inclinée (13).

5. Cathéter pour conduit hépatique commun selon la revendication 1, dans lequel, dans le perforateur (3), une surface circonférentielle extérieure est pourvue d'une section en coin (3b) et la section en coin (3b) est pourvue d'une partie découpée (3c) saillant dans le sens de la longueur.

6. Cathéter pour conduit hépatique commun selon la revendication 5, dans lequel, dans la partie découpée (3c), un angle décrit par la partie découpée (3c) est compris entre 30° et 45°, vu depuis une surface découpée coupée dans un sens à angle droit par rapport à la ligne centrale (O) dans le sens de la longueur.
